# EUROPEAN PATENT APPLICATION

(11) **EP 4 803 019 A1**
(43) Date of publication of application: **09.09.2026**
(21) Application number: 26162561.0
(22) Date of filing: 05.03.2026
(51) Int. Cl.: A61B 17/72

(54) **HUMERAL INTRAMEDULLARY NAIL**

(30) Priority: 05.03.2025 US 202563767275 P
(71) Applicant: Stryker European Operations Limited, Carrigtwohill T45H X08 (IE)
(72) Inventor: KLEINER, Oliver, 24223 Schwentinental (DE); NIEBUHR, Milan, 24116 Kiel (DE)
(74) Representative: Maiwald GmbH

(57) **Abstract**

A humeral intramedullary nail (100) may include a length extending between first and second ends (121, 122), wherein the length includes a longitudinal axis (125). The humeral intramedullary nail may also include a distal portion adjacent (123) to the first end (121), a proximal portion (124) adjacent to the second end (122), and a first oblong distal aperture (101) formed through the distal portion (123). The first distal aperture may be configured to receive a first fastening member in a first direction transverse to the longitudinal axis. The humeral intramedullary nail may further include a second distal aperture (102) formed through the distal portion (123). The second distal aperture may be closer to the first end than the first oblong distal aperture and configured to receive a second fastening member in a second direction transverse to the longitudinal axis. The humeral intramedullary nail may further include a plurality of proximal apertures (111, 112, 113, 114, 115) formed through the proximal portion (124).

## Description

### BACKGROUND OF THE INVENTION

The skeletal system includes many different types of bones, which in turn require different orthopedic treatment protocols. For instance, the repair of fractures of long bones (*e.g*., the femur and humerus) often makes use of intramedullary nails. These implants are elongate structures designed to fit within the medullary canal or space inside the diaphysis of the bone (*i.e*., center of the bone) to stabilize the fracture. Screws are often utilized in conjunction with an intramedullary nail to fix it in place within the bone. While often inserted through the bone and into the distal and proximal portions of the nail, different bones and nail sizes require different screw placement.

For instance, intramedullary nails for use in the humerus require specific screw placement so as to avoid medical complications such as unwanted contact with nerves, blood vessels, tissues such as tendons, other biological matter, and the like. Of particular concern in the humerus is the biceps tendon and radialis nerve. Moreover, anatomy differs among patients and requires some flexibility in the placement of the screws, while still maintaining optimal nail stability.

Therefore, there exists a need for an improved intramedullary nail, particularly for use in the humerus.

### BRIEF SUMMARY OF THE INVENTION

Although not necessarily limited to a specific nail construct, the present disclosure describes examples of short humeral intramedullary nails which are configured to receive specifically placed fastening members therein. Such fastening members are individually inserted into different apertures along a length of the intramedullary nail. The plurality of apertures include various sized and shaped apertures along various planes of the intramedullary nail. Such aperture configuration of the intramedullary nail is designed to increase stability by providing more locking options, as well as mitigating the risk of interference with biological matter such as the biceps tendon and the radialis nerve.

In accordance with an aspect of the present disclosure, a humeral intramedullary nail includes a length, a distal portion, a proximal portion, a plurality of distal apertures, and a plurality of proximal apertures. The length extends between first and second ends and includes a longitudinal axis. The distal portion is adjacent to the first end. The proximal portion is adjacent to the second end. The plurality of distal apertures includes a first oblong distal aperture and a second distal aperture. The first oblong distal aperture is formed through the distal portion and is configured to receive a first fastening member in a first direction transverse to the longitudinal axis. The second distal aperture is formed through the distal portion and is closer to the first end than the first oblong distal aperture and is configured to receive a second fastening member in a second direction transverse to the longitudinal axis. The plurality of proximal apertures are formed through the proximal portion.

In other embodiments of this aspect, the humeral intramedullary nail may include a third distal aperture formed through the distal portion. The third distal aperture is configured to receive a third fastening member in a third direction transverse to the longitudinal axis and is farther from the first end than the first and second apertures. The second and third fastening members may be threaded screws. The first and second directions may be rotated between 1 to 89 degrees. The plurality of proximal apertures may be transverse to the longitudinal axis. The plurality of proximal apertures may be configured to receive fastening members.

In accordance with another aspect of the present disclosure, a humeral intramedullary nail includes a first end, a second end, a distal portion, a proximal portion, a length, and a plurality of apertures. The second end extends from the first end. The distal portion is adjacent to the first end. The proximal portion is adjacent to the second end. The length extends between the first and second end and has a longitudinal axis. The plurality of apertures extends through the length, where the plurality of apertures are transverse to the longitudinal axis and are configured to each receive a fastening member. The plurality of apertures includes a first, second, and third distal aperture. The second distal aperture being the closest of the plurality of apertures to the first end and rotated about the longitudinal axis of the nail between 1 to 89 degrees with respect to the first distal aperture.

In other embodiments of this aspect, the third distal aperture may be the second closest of the plurality of apertures to the first end and the first distal aperture is the third closest of the plurality of apertures to the first end. The third distal aperture may be oblong. The fastening member may be a threaded screw. The plurality of apertures may include a first, second, third, fourth, and fifth proximal apertures. The first proximal aperture being the closest of the plurality of apertures to the second end. The second proximal aperture being between the first proximal aperture and third proximal aperture. The fourth proximal aperture being between the third proximal aperture and fifth proximal aperture. The fifth proximal aperture being the fifth closest of the plurality of apertures to the second end. The second proximal aperture may be rotated 74 degrees about the longitudinal axis with respect to the first proximal aperture. The third proximal aperture may be rotated 325 degrees about the longitudinal axis with respect to the first proximal aperture. The fourth proximal aperture may be rotated 290 degrees about the longitudinal axis with respect to the first proximal aperture. The fifth proximal aperture may be rotated 355 degrees about the longitudinal axis with respect to the first proximal aperture.

In accordance with another aspect of the present disclosure, a humeral nail includes a first end, a second end, a length, and a plurality of apertures. The first end has a distal portion adjacent to the first end. The second end has a proximal portion adjacent to the second end. The length being between the first and second ends and includes a longitudinal axis. The plurality of apertures extending through the length and are transverse to the longitudinal axis and are configured to each receive a fastening member. The plurality of apertures including a first, second, and third distal aperture. The second distal aperture is the closest of the plurality of apertures to the first end along the longitudinal axis and is rotated about the longitudinal axis with respect to the first distal aperture by at least a degree.

In other embodiments of this aspect, the plurality of apertures may include a third distal aperture and is the second closest of the plurality of apertures to the first end. The third distal aperture may be oblong. The fastening member may be a threaded screw. The plurality of apertures may include a first, second, third, fourth, and fifth proximal aperture. The first proximal aperture may be the closest of the plurality of apertures to the second end. The second proximal aperture may be between the first proximal aperture and third proximal aperture. The fourth proximal aperture may be between the third proximal aperture and fifth proximal aperture. The fifth proximal aperture may be the fifth closest of the plurality of apertures to the second end. The first, second, third, fourth, and fifth proximal apertures may be rotated about the longitudinal axis with respect to each other. The second proximal aperture may be rotated 74 degrees about the longitudinal axis with respect to the first proximal aperture. The third proximal aperture may be rotated 325 degrees about the longitudinal axis with respect to the first proximal aperture, the fourth proximal aperture may be rotated 290 degrees about the longitudinal axis with respect to the first proximal aperture. The fifth proximal aperture may be rotated 355 degrees about the longitudinal axis with respect to the first proximal aperture.

### BRIEF DESCRIPTION OF THE DRAWINGS

The features, aspects, and advantages of the present invention will become better understood with regard to the following description, appended claims, and accompanying drawings in which:
FIG. 1 is an illustration of a side view of an intramedullary humeral nail according to an embodiment of the present disclosure.
FIG. 2 is a side view of the intramedullary humeral nail of FIG. 1 with fastening members inserted therein.
FIG. 3A is another side view of the intramedullary humeral nail of FIG. 1 without fastening members inserted therein.
FIG. 3B is another side view of the intramedullary humeral nail of FIG. 1 with fastening members inserted therein.
FIG. 4A is another side view of the intramedullary humeral nail of FIG. 1 without fastening members inserted therein.
FIG. 4B is another side view of the intramedullary humeral nail of FIG. 1 with fastening members inserted therein.
FIG. 5 is a cross-sectional view of the intramedullary humeral nail of FIG. 1 without fastening members inserted therein.
FIG. 6A is another side view of the intramedullary humeral nail of FIG. 1 with proximal fastening members inserted therein.
FIG. 6B is another side view of the intramedullary humeral nail of FIG. 1 with distal fastening members inserted therein.
FIG. 6C is another side view of the intramedullary humeral nail of FIG. 1 with proximal and distal fastening members inserted therein.
FIG. 7 is a perspective enlarged view of a proximal portion of the intramedullary humeral nail of FIG. 1.
FIG. 8 is another perspective view of the proximal portion of FIG. 7.
FIG. 9 is a top view of the proximal portion of FIG. 7.
FIG. 10A is another top view of the proximal portion of FIG. 7 with proximal fastening members inserted therein.
FIG. 10B is another top view of the proximal portion of FIG. 7 with distal fastening members inserted therein.
FIG. 10C is another top view of the proximal portion of FIG. 7 with proximal and distal fastening members inserted therein.
FIG. 11 is a bottom view of a distal portion of the intramedullary humeral nail of FIG. 1.
FIG. 12 is a perspective view of a distal portion of the intramedullary humeral nail of FIG. 1.
FIG. 13 is a side view of the intramedullary humeral nail of FIG. 1 with fastening members inserted therein inserted within a humerus (shown partially transparent).

### DETAILED DESCRIPTION

In describing the preferred embodiments of the inventions, specific terminology will be used for the sake of clarity. However, the inventions are not intended to be limited to any specific terms used herein, and it is to be understood that each specific term includes all technical equivalents, which operate in a similar manner to accomplish a similar purpose. In the drawings and in the description which follows, the term "proximal" means closer to the heart and the term "distal" means more distant from the heart. The term "anterior" means towards the front part of the body or the face and the term "posterior" means towards the back of the body. The term "medial" means toward the midline of the body and the term "lateral" means away from the midline of the body. It should be further understood that intramedullary nails described herein can include a left nail and a right nail. Embodiments described herein should not be construed as to be limited to only a left or right nail. Rather, an embodiment discussing, for example, a left nail, should be equally construed to include a right nail as well.

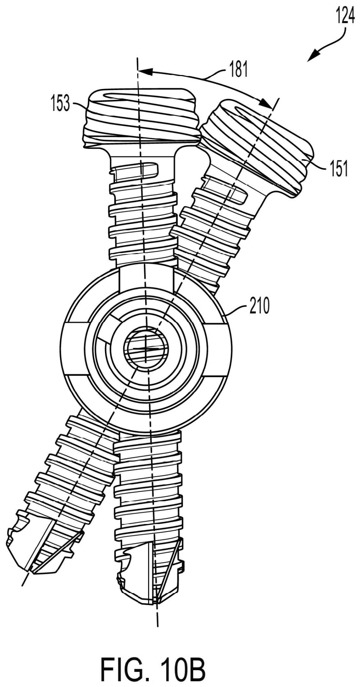

The various disclosed embodiments include a system and method for an intramedullary nail and insertion of screws thereof. The intramedullary nail includes apertures along a length of the nail, with the apertures being configured to receive fastening members to stabilize a desired bone. The apertures include proximal apertures adjacent to a proximal portion of the intramedullary nail and distal apertures adjacent to a distal portion of the intramedullary nail. The apertures are transverse to a longitudinal axis of the intramedullary nail and certain of them are rotated about the longitudinal axis with respect to each other. Specifically, the distal apertures include three distal apertures in which a first distal aperture is rotated to be in a different plane than the other two distal apertures. As shown, in *e.g*., FIG 10B, the first distal aperture is rotated at a first angle 181 of 30 degrees with respect to the other distal apertures, but can be rotated between at least 1 and 89 degrees, 91 and 179 degrees, 181 and 269 degrees, and 271 and 359 degrees. The distal apertures further include a second distal aperture which is an oblong distal aperture. The length of the oblong distal aperture allows for a fastening member to be inserted at various lengths along the aperture allowing for dynamic locking. The proximal apertures include five proximal apertures in which all five are rotated to be in different planes from one another.

The configuration of varying aperture placements along the length of the intramedullary nail as well as the oblong shape of the second distal aperture allows for greater bone stability and a reduction of risk of interference with biological matter such as the biceps tendon and radialis nerve. This is advantageous as it allows for more fastening members to be inserted into the intramedullary nail to stabilize a bone while simultaneously mitigating the risk of undesirable contact with biological matter. Specifically, the variation in placement of the first distal aperture with respect to the oblong distal aperture allows fastening members to avoid unwanted contact with biological matter such as nerves, both during insertion and after being placed. Further, the oblong distal aperture achieves a similar result, as a fastening member can be inserted into the oblong distal aperture at various points depending on the location of biological matter in any given patient.

The insertion of distal fastening members can be targeted to either the distal oblong aperture or the distal round apertures depending on the fracture. Particularly, an unstable fracture may have the intramedullary nail configuration focus on stability in which fastening members can be inserted into at least the round distal apertures. For a fracture where stability is less critical, dynamization may be prioritized such that no fastening members are inserted into the round distal apertures and instead, the oblong distal aperture has a fastening member inserted therein. According to an embodiment, fastening members can be inserted into both the round distal apertures and the oblong distal aperture wherein the fastening members fastened to the round apertures may be removed after some time, such that only the oblong aperture fastening member is attached, causing dynamization to occur. It should be appreciated that the intramedullary humeral nail may also include a second oblong distal aperture. In an embodiment, the second oblong distal aperture is rotated as to be in a different plane than the first oblong distal aperture. This configuration allows for more stability during dynamization than a one oblong distal aperture configuration as it further limits the degree of freedom the intramedullary nail has within a patient.

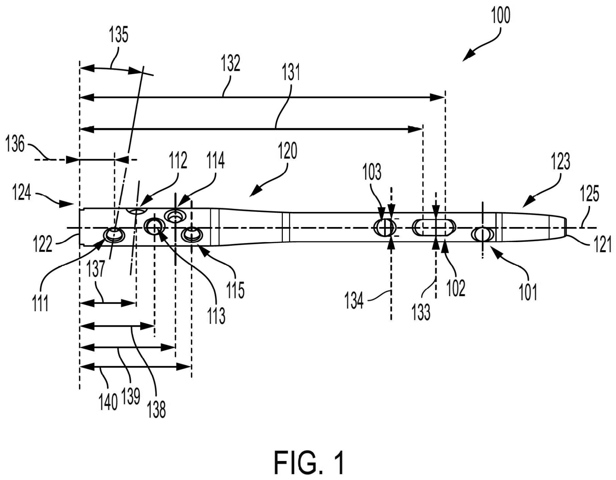

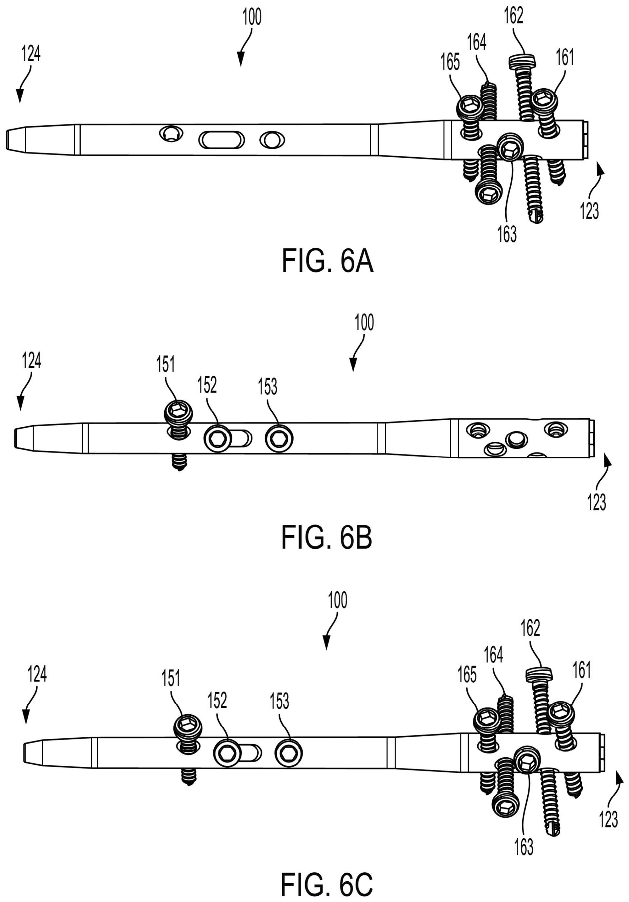

Generally, FIGs. 1-13 show an intramedullary nail 100 with different configurations of fastening members inserted into apertures.

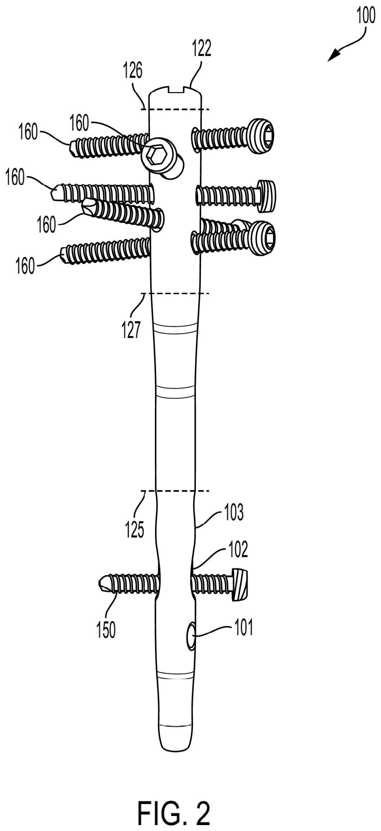

FIG. 1 is an illustration of a schematic side view of intramedullary nail 100 according to an embodiment. Intramedullary nail 100 depicted includes a shaft 120 extending about a longitudinal axis 125. Shaft 120 extends between a first or distal end 121 and a second or proximal end 122. Shaft 120 includes a distal portion 123 adjacent to first end 121 and a proximal portion 124 adjacent to second end 122, wherein each of the distal and proximal portions have several apertures. In an embodiment, the apertures are formed through shaft 120 transverse to the longitudinal axis 125. Each of the apertures are configured to receive a fastening member such as, but not limited to, screws (as shown in *e.g*., FIG. 2), nails, bolts, anchors, rivets, any other fasteners, a combination thereof, and the like. Each of the fastening members pass entirely through nail 100 in a direction transverse to longitudinal axis 125. Although it is contemplated to include apertures of differing sizes and shapes, circular apertures 101, 103, 111-115 each have a diameter 134 of approximately 4.5 millimeters, with a tolerance of approximately +0.3 millimeters. The circular apertures are configured to enable static locking. Although it could be various sizes and shapes, the length of shaft 120 between the first end 121 and the second end 122 is approximately 130 millimeters.

Distal portion 123 of shaft 120 includes a first distal aperture 101, a second distal aperture 102, and a third distal aperture 103. As shown, the direction of the first distal aperture 101 is rotated from the other distal apertures. This rotation can be by at least one degree with respect to the other distal apertures, but as shown is offset by thirty degrees with respect to the other distal apertures. First distal aperture 101 is rotated from the other distal apertures to at least have fastening members avoid contact with nerves such as, but not limited to, radial, median, ulnar, musculocutaneous, posterior, lateral, and the like. First distal aperture 101 may also be rotated to avoid other unwanted contact or mitigate the chance of unwanted contact such as with arteries, veins, other nerves, tissues, tendons, other biological matter, medical implants, a combination thereof, and the like. Moreover, having apertures in different planes allows for greater stability by providing more locking options and increases the strength against bending. As shown, first distal aperture 101 is the closest of the plurality of apertures to first end 121, second distal aperture 102 is the second closest of the plurality of apertures to first end 121, and third distal aperture 103 is the third closest of the plurality of apertures to first end 121.

Second distal aperture 102 is an oblong aperture such as, but not limited to a slot. The oblong aperture is configured to enable dynamization. Particularly, the oblong nature of second distal aperture 102 allows for a fastening member to be dynamically positioned such the intramedullary nail can move and the fracture can settle while torsional stability is maintained. As a non-limiting example, a fastening member can be inserted near a distal portion of oblong aperture 102 such that as the bone compresses the fastening member moves proximally within oblong aperture 102.

A first length 131 measured between second end 122 and the center of a first curve of second distal aperture 102 is approximately 91.5 millimeters. A second length 132 measured between second end 122 and the center of a second curve of second distal aperture 102 is approximately 97.5 millimeters. The width 133 of second distal aperture 102 is approximately 4.1 millimeters and the tolerance of width 133 is approximately +0.1 millimeters. The oblong nature of second distal aperture 102 permits a fastening member to be inserted into the aperture at various points along width 133.

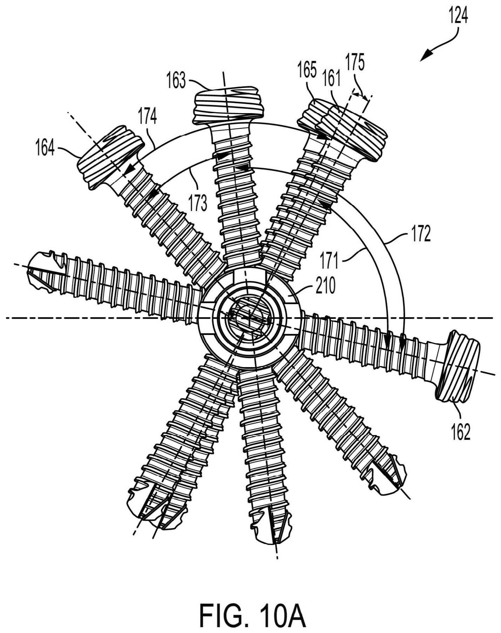

Proximal portion of shaft 120 includes a first proximal aperture 111, a second proximal aperture 112, a third proximal aperture 113, a fourth proximal aperture 114, and a fifth proximal aperture 115. These proximal apertures are rotated about the longitudinal axis 125 with respect to each other. In particular, first proximal aperture 111 is positioned at 29 degrees, second proximal aperture 112 is positioned at 103 degrees, third proximal aperture 113 is positioned at 354 degrees, fourth proximal aperture 114 is positioned at 319 degrees, and fifth proximal aperture 115 is positioned at 24 degrees. Alternatively, the positioning of the plurality of proximal apertures can be as shown in FIG. 10A, such that second proximal aperture 112 is rotated a first angle 171 of 74 degrees about longitudinal axis 125 with respect to first proximal aperture 111 in a first direction; third proximal aperture 113 is rotated a second angle 172 of 109 degrees about longitudinal axis 125 with respect to second proximal aperture 112 in a second direction opposite the first direction; fourth proximal aperture 114 is rotated a third angle 173 of 35degrees about longitudinal axis 125 with respect to third proximal aperture 113 in the second direction; fifth proximal aperture 115 is rotated a fourth angle 174 of 65 degrees about longitudinal axis 125 with respect to fourth proximal aperture 114 in the first direction; and first proximal aperture 111 is rotated a fifth angle 175 of 5 degrees about longitudinal axis 125 with respect to fifth proximal aperture 115 in the first direction. As shown in the embodiment of FIG. 1, the first direction is clockwise. In other embodiments, the first direction is counterclockwise.

First proximal aperture 111 is tilted by an angle 135 as to not be perpendicular to the longitudinal axis 125. In some embodiments, the angle 135 is approximately 5 degrees such that the angles between the longitudinal axis 125 and the axis of the proximal aperture 111 through the shaft 120 are 85 degrees and 95 degrees. In a further embodiment, the second proximal aperture 112 also is tilted by an angle 135 wherein the angle 135 is 5 degrees.

As shown, first proximal aperture 111 is the closest of the plurality of apertures to second end 122, second proximal aperture 112 is the second closest of the plurality of apertures to second end 122, third proximal aperture 113 is the third closest of the plurality of apertures to second end 122, fourth proximal aperture 114 is the fourth closest of the plurality of apertures to second end 122, and fifth proximal 115 aperture is the fifth closest of the plurality of apertures to second end 122. This results in fifth proximal aperture 115 being closer to second end 122 than any of the distal apertures. Put another way, fifth proximal aperture 115 is farther from first end 121 than any of the distal apertures.

A distance 136 between second end 122 and the center of first proximal aperture 111 is approximately 9.5 millimeters. A distance 137 between second end 122 and the center of second proximal aperture 112 is approximately 15 millimeters. A distance 138 between second end 122 and the center of third proximal aperture 113 is approximately 20 millimeters. A distance 139 between second end 122 and the center of fourth proximal aperture 114 is approximately 25.5 millimeters. A distance 140 between second end 122 and the center of fifth proximal aperture 115 is approximately 30 millimeters.

FIG. 2 is a first side view of an intramedullary nail with fastening members inserted therein 100 according to an embodiment.

The plurality of apertures are configured to each receive a fastening member. The fastening members are configured to be positioned within each of the plurality of apertures as to intersect longitudinal axis 125. As shown, the fastening members are positioned substantially equidistant within intramedullary nail 100 such that the length of the fastening member exposed from each opening of an aperture is similar. However, the fastening members are not limited to this configuration, the fastening members can also be positioned at various other lengths along the length of the fastening member within the apertures. For example, a patient's humerus might have varied or nonuniform thickness requiring the fastening members to be positioned non-equidistantly within their respective apertures as to provide desirable support. Furthermore, the varying placement of the fastening members within the apertures provides for a wider variety of support configurations depending on the fracture (*e.g*., type of fracture, severity of fracture, angle of fracture, etc.). The size of the fastening members themselves can also vary. Specifically, the size of all fastening members can vary relative to the patient's humerus (*e.g*., size, age, gender, etc.) as well as varying relative to one another (*i.e*., proximal versus distal fastening members).

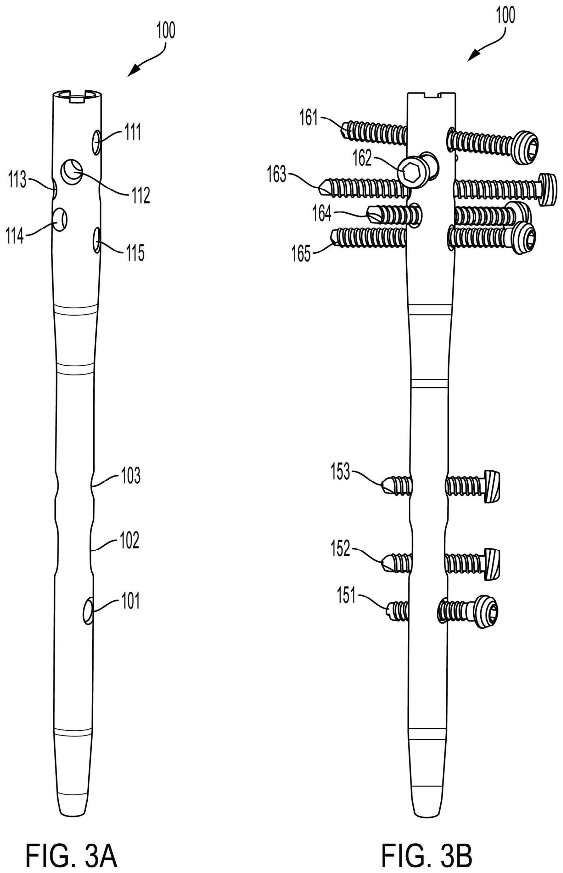

The fastening members can include screws such as distal screw 150 and proximal screws 160. Intramedullary nail 100 can include various configurations of screws such as shown in *e.g*., FIG. 3B.

Distal screw 150 is configured to be received by any of the distal apertures such as the second distal aperture 102. Distal screw 150 may be utilized for each of the plurality of distal apertures such that all, some, or none of the distal apertures have distal screw 150 inserted therein. In an embodiment, distal screw 150 can be received by any of the proximal apertures.

Proximal screw 160 is configured to be received by any of the proximal apertures. Proximal screw 160 may be utilized for each of the plurality of proximal apertures such that all, some, or none of the proximal apertures have the proximal screw 160 inserted therein. Up to three of the inserted proximal screws 160 are configured to support fixation of the greater tuberosity, up to one of the inserted proximal screws 160 is configured to support fixation of the lesser tuberosity, and up to one of the proximal screws 160 is configured to support fixation of the calcar. It should be appreciated that proximal screws 160 may be inserted in any other configuration or combination. As a non-limiting example, two of three proximal screws 160 are utilized to support the greater tuberosity, zero of one proximal screws 160 are utilized to support the lesser tuberosity, and one of one proximal screws 160 are utilized as a calcar screw. In an embodiment, proximal screw 160 can be received by any of the distal apertures.

The oblong nature of distal aperture 102 allows for distal screw 150 to be positioned at various lengths along the slot length. Such variability in placement allows for a desirable placement within a patient, as the anatomical structure of the humerus for each patient can vary. Further, the oblong shape of distal aperture 102 allows for a fastening member, such as distal screw 150, to be inserted in a dynamic position such that the fractured humerus can compress while the distal screw 150 moves in a proximal direction within the oblong aperture.

The variability in terms of quantity of distal screws 150 and proximal screws 160 used for intramedullary nail 100 allows for more desirable configurations. Specifically, unwanted contact with biological matter can be avoided while still providing support by inserting screws into apertures which are not at risk of unwanted contact.

Shaft 120 includes a distal cross-section 125 and a proximal cross-section 126. Distal cross-section 125 is adjacent to first end 121 and distal portion 123 while proximal cross-section 126 is adjacent to second end 122 and proximal portion 124. Distal cross-section 125 has a diameter of approximately 8 millimeters and proximal cross-section 126 has a diameter of approximately 10 millimeters. Shaft 120 includes a middle cross-section 127 between distal cross-section 125 and proximal cross-section 126. Middle cross-section 127 has a tapering diameter from the diameter of proximal cross-section 126 to the diameter of distal cross-section 125.

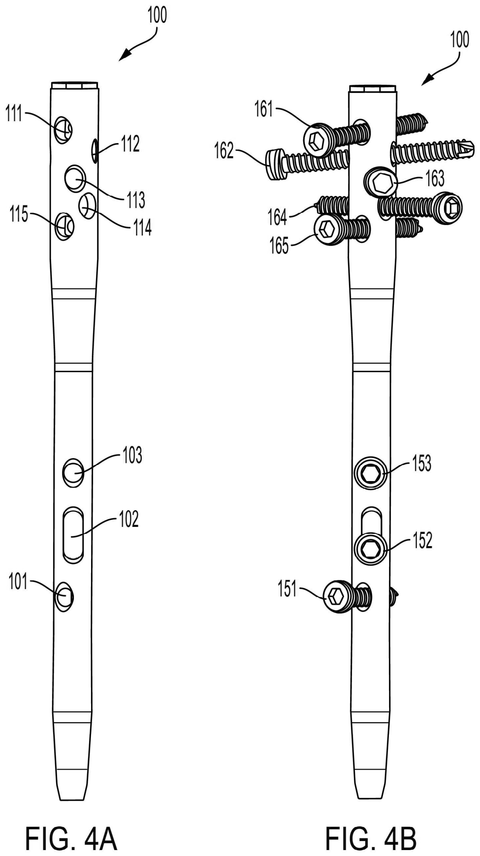

FIGs. 3A-B are different side views of intramedullary nail 100, with (FIG. 3B) and without (FIG. 3A) screws inserted therein. As shown in FIG. 3B, intramedullary nail 100 has all distal screws 150 and proximal screws 160 inserted placed therethrough. Intramedullary nail 100 includes a first proximal screw 161, a second proximal screw 162, a third proximal screw 163, a fourth proximal screw 164, a fifth proximal screw 165, a first distal screw 151, a second distal screw 152, and a third distal screw 153. Proximal screws 161-165 are inserted within respective proximal apertures 111-115 such that first proximal aperture 111 receives first proximal screw 161 and so forth. Likewise, distal screws 151-153 are inserted within respective distal apertures 101-103 such that first distal aperture 101 receives first distal screw 151 and so forth. The proximal and distal screws can also be inserted in any other configuration such that the humerus is supported while avoiding unwanted risk of contact with certain biological matter. As a non-limiting example, first proximal aperture 111 can receive *e.g*., third proximal screw 163, fifth proximal screw 165, or first distal screw 151, etc. FIGs. 4A-B are similar to FIGs. 3A-B taken about a different side of intramedullary nail 100.

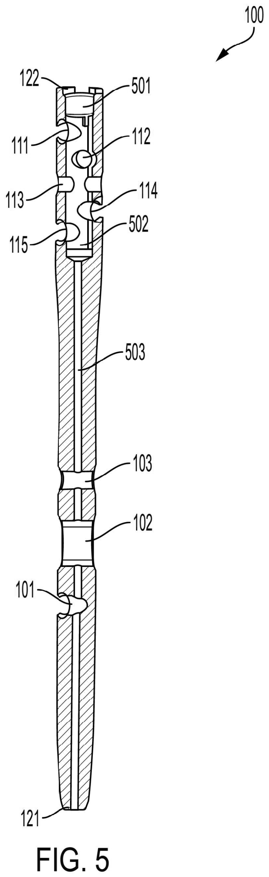

FIG. 5 is a cross-sectional view of intramedullary humeral nail 100 according to an embodiment. As shown, intramedullary nail 100 includes a first bore 501, a second bore 502, and a third bore 503. First bore 501 extends from an opening of second end 122 along a portion of the longitudinal length of intramedullary nail 100. Second bore 502 extends from an end of first bore 501 opposite the end extending from the opening of second end 122. Second bore 502 extends at least along a portion of the longitudinal length of intramedullary nail 100 wherein the plurality of proximal apertures are defined. Third bore 503 extends from an end of second bore 502 opposite the end adjacent to first bore 501, to an opening of the first end 121. Third bore 503 extends at least along a portion of the longitudinal length of intramedullary nail 100 wherein the plurality of distal apertures are defined.

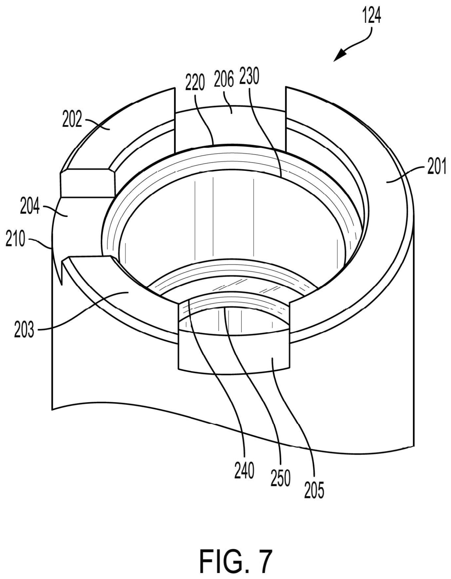

FIG. 7 is a perspective view of a proximal portion of intramedullary humeral nail 100. As shown, proximal portion includes a plurality of circumferences, a plurality of ridges, and a plurality of depressions. The plurality of circumferences includes a first circumference 210, a second circumference 220, a third circumference 230, a fourth circumference 240, and a fifth circumference 250. The plurality of ridges includes a first ridge 201, a second ridge 202, and a third ridge 203. The plurality of depressions includes a first depression 204, a second depression 205, and a third depression 206. The plurality of ridges and depressions are positioned between first circumference 210 and second circumference 220. The plurality of ridges and depressions together form an annulus-like shape. Individually, first ridge 201 is between second depression 205 and third depression 206 along a centerline of the annulus-like shape, second ridge 202 is between first depression 204 and third depression 206 along the centerline, and third ridge 203 is between the first depression 204 and second depression 205 along the centerline.

First circumference 210 is the circumference for a majority of an outer surface of distal portion 123 of shaft 120. Third circumference 230 is the inner circumference of a first thickness of shaft 120 that extends inwardly from first circumference 210 and is the circumference for a majority of a surface of first bore 501. Fifth circumference 250 is the circumference for a majority of a surface of second bore 502 and is the inner circumference of a second thickness of shaft 120 that extends inwardly from first circumference 210 and which goes further inward than third circumference 230. Sixth circumference 260 is the circumference for a majority of third bore 503 and is the inner circumference of a varying thickness which extends from an outer surface of a center and distal portion 123 of shaft 120.

Second circumference 220 is between first circumference 210 and third circumference 230. Between second circumference 220 and third circumference 230 is a tapering length such as those found on countersink holes. Fourth circumference 240 is between third circumference 230 and fifth circumference 250 and includes another tapering length. Ridges 201-203 each have rounded edges adjacent to the first circumference 210 as well as the second circumference 220.

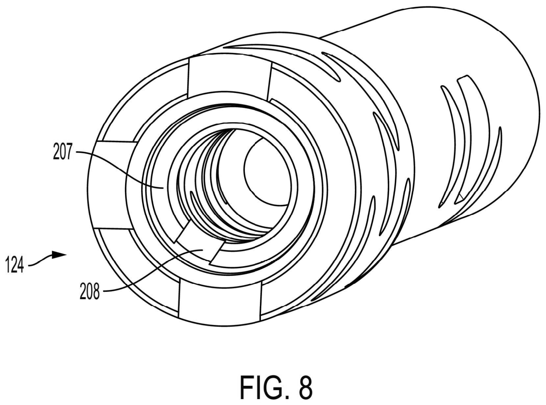

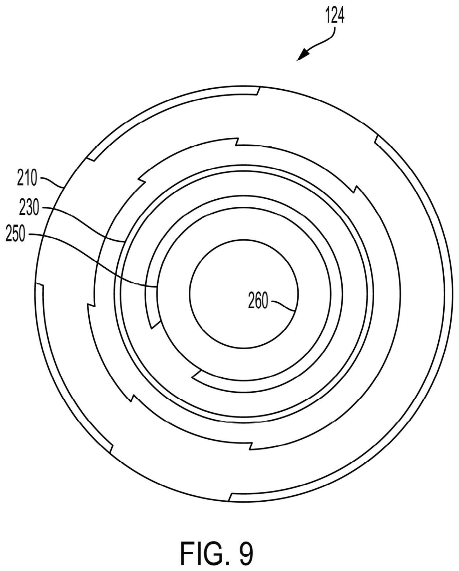

FIG. 8 is another perspective view of a proximal portion of an intramedullary humeral nail 100 according to an embodiment. The perspective view of the proximal portion depicts a fourth ridge 207 and a fourth depression 208. Fourth ridge 207 and fourth depression 208 are positioned between third circumference 230 and fifth circumference 250. Together, fourth ridge 207 and fourth depression 208 form an annulus-like shape. FIG. 9 is a top view of proximal portion 124.

First bore 501 is configured to receive an end cap following insertion of intramedullary nail 100 within a humerus. The plurality of ridges and the plurality of depressions are configured to receive an attachment portion of an insertion device such that during insertion of intramedullary nail 100, intramedullary nail 100 is rotationally constrained with respect to the insertion device. Third bore 503 is configured to receive a guide wire during the insertion process of intramedullary nail 100.

Second bore 502 is configured to receive at least one polymeric sleeve. The polymer sleeve is configured to encase any fastening members inserted into the proximal apertures to secure the inserted fastening members to intramedullary nail 100. Specifically, the polymer sleeve helps to inhibit inserted fastening members from backing out of the intramedullary nail 100. In an embodiment, no polymer sleeves are utilized.

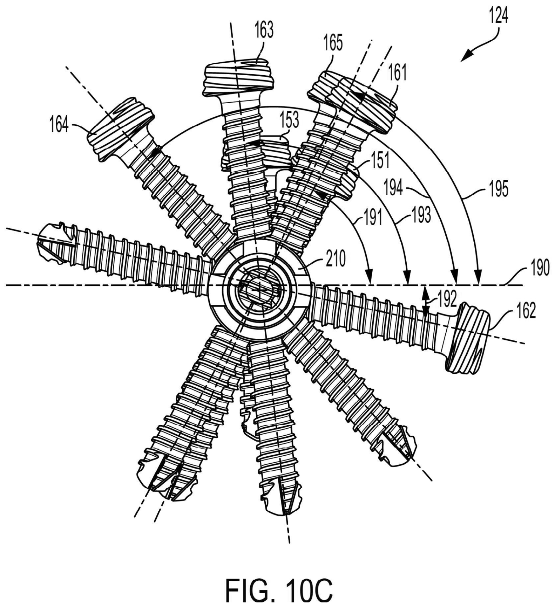

FIG. 10C is another top view of the proximal portion of FIG. 7 with proximal and distal fastening members inserted therein. Particularly, FIG. 10C shows the angle of proximal fastening members with respect to a centerline 190 along second depression 205 and third depression 206. First angle 191 depicts the angle between first proximal screw 161 and centerline 190, first angle 191 being 61 degrees. Second angle 192 depicts the angle between second proximal screw 162 and centerline 190, second angle 192 being 23 degrees. Third angle 193 depicts the angle between third proximal screw 163 and centerline 190, third angle 193 being 96 degrees. Fourth angle 194 depicts the angle between fourth proximal screw 164 and centerline 190, fourth angle 194 being 131 degrees. Fifth angle 195 depicts the angle between fifth proximal screw 165 and centerline 190, fifth proximal angle 195 being 66 degrees. Of course, in other embodiments, these angle values can differ from that specifically shown in the figures.

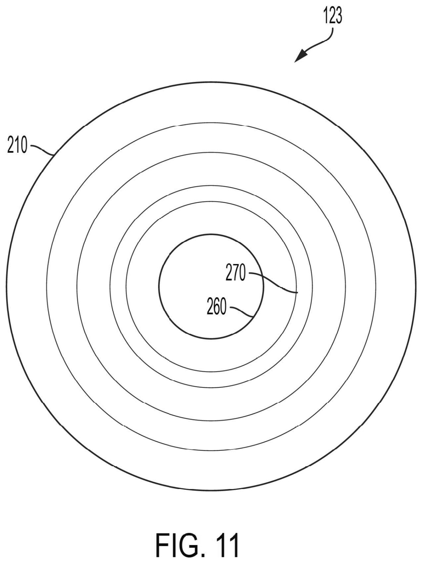

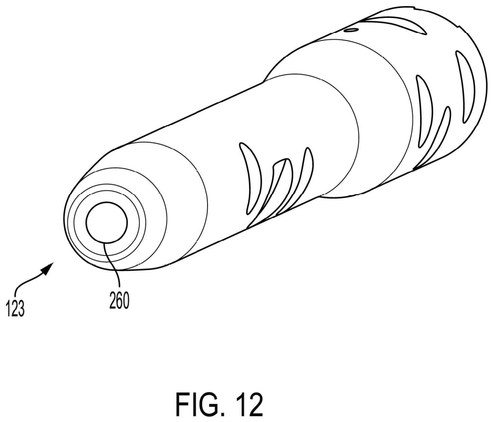

FIG. 11 is a bottom view of distal portion 123 of intramedullary humeral nail 100 and FIG. 12 is a perspective view of the distal portion. As shown, distal portion 123 includes a seventh circumference 270. Seventh circumference 270 is the circumference of the outer surface of shaft 120 at first end 121. Seventh circumference 270 has a rounded edge.

A method of repairing a humeral fracture according to the present disclosures includes inserting a humeral nail into an intramedullary canal of a humerus from a proximal end thereof. Following an incision, an entry point is made (*e.g*., with an awl) and the humerus is reamed. Subsequently, the intramedullary nail is inserted into the humerus. According to an embodiment, intramedullary nail 100 is inserted into the humerus with a target device, a nail holding screw, and a slotted hammer. The target device is configured to have guided locking of the distal and proximal screws to be inserted into intramedullary nail 100. In another embodiment, the fastening members are inserted utilizing a freehand technique. In yet another embodiment, the fastening members are inserted utilizing an antegrade technique. All fastening members are configured to be inserted laterally to mitigate the risk of interference with the biceps tendon and the radialis nerve. Following the insertion of the fastening members, an end cap is inserted at second end 122 of intramedullary nail 100. The end cap and the second end have a pitch thread of M7.

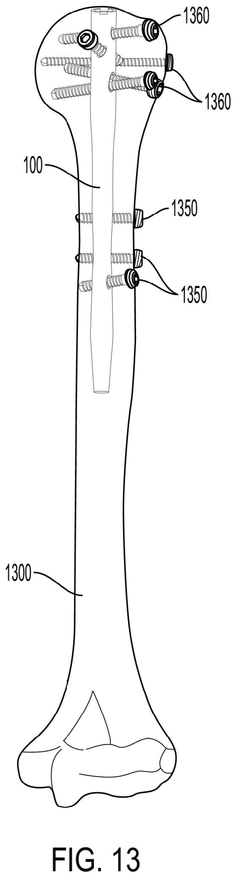

FIG. 13 is a side view of an intramedullary humeral nail shown with fastening members inserted within a humerus. As shown, intramedullary nail 100 is positioned within a humerus 1300 with proximal screws 1360 and distal screws 1350 inserted therein. Proximal screws 1360 and distal screws 1350 are configured to be substantially placed within bone material. The heads of proximal screws 1360 and distal screws 1350 are configured to be positioned outside of humerus 1300.

The fastening members are configured to provide fixation of bone fragments such as, but not limited to, the lesser tuberosity, the greater tuberosity, the calcar, the head, the bicipital groove, the surgical neck, the like, and any combination thereof. As shown, distal screws 1360 are smaller in length than proximal screws 1360 to accommodate for the general anatomy of a humerus wherein the proximal portion (*i.e*., head and neck) has a greater radius than a more center portion (*i.e*., tuberosity). However, the present disclosure is not limited to this configuration. In some embodiments, distal screws 1350 can be longer than proximal screws 1360 and proximal screws 1360 can be shorter than distal screws 1350. In yet a further embodiment, distal and proximal screws can be interchangeably placed in any configuration. As a non-limiting example, the five proximal apertures can include several distal screws 1350 and several proximal screws 1360.

## Claims

1. A humeral intramedullary nail comprising:
a length extending between first and second ends, wherein the length includes a longitudinal axis;
a distal portion adjacent to the first end;
a proximal portion adjacent to the second end;
a first distal aperture formed through the distal portion, the first distal aperture being configured to receive a first fastening member in a first direction transverse to the longitudinal axis;
a second distal aperture formed through the distal portion, the second distal aperture being closer to the first end than the first distal aperture and configured to receive a second fastening member in a second direction transverse to the longitudinal axis, the second direction being different than the first direction; and
a plurality of proximal apertures formed through the proximal portion.

2. The humeral intramedullary nail of claim 1, wherein the first distal aperture is oblong.

3. The humeral intramedullary nail of any one of claims 1 and 2, further comprising a third distal aperture formed through the distal portion, the third distal aperture being configured to receive a third fastening member in a third direction transverse to the longitudinal axis and being farther from the first end than the first and second distal apertures.

4. The humeral intramedullary nail of any one of claims 1 to 3, wherein the second and third fastening members are threaded screws.

5. The humeral intramedullary nail of any one of claims 1 to 4, wherein the second direction of the second distal aperture is rotated 30 degrees with respect to one of the first direction or the third direction.

6. The humeral intramedullary nail of any one of claims 1 to 4, wherein the second direction of the second distal aperture is rotated 30 degrees with respect to the first direction of the first distal aperture.

7. The humeral intramedullary nail of any one of claims 1 to 6, wherein the first direction is rotated 0 degrees with respect to the third direction of the third distal aperture.

8. The humeral intramedullary nail of any one of claims 1 to 7, wherein the first and second directions are rotated between 1 degree to 89 degrees.

9. The humeral intramedullary nail of any one of claims 1 to 8, wherein the plurality of proximal apertures are transverse to the longitudinal axis.

10. The humeral intramedullary nail of any one of claims 1 to 9, wherein the second distal aperture is the closest aperture among the plurality of proximal apertures and the first, second, and third distal apertures to the first end, the second distal aperture being rotated about the longitudinal axis between 1 degree to 89 degrees with respect to the first distal aperture.

11. The humeral intramedullary nail of any one of claims 1 to 10, wherein the plurality of proximal apertures further include first, second, third, fourth, and fifth proximal apertures, the first proximal aperture being the closest of the plurality of proximal apertures to the second end, the second proximal aperture being between the first proximal aperture and third proximal aperture, the fourth proximal aperture being between the third proximal aperture and fifth proximal aperture, the fifth proximal aperture being the fifth closest of the plurality of proximal apertures to the second end.

12. The humeral intramedullary nail of any one of claims 1 to 11, wherein the second proximal aperture is rotated 74 degrees about the longitudinal axis with respect to the first proximal aperture, the third proximal aperture is rotated 325 degrees about the longitudinal axis with respect to the first proximal aperture, the fourth proximal aperture is rotated 290 degrees about the longitudinal axis with respect to the first proximal aperture, the fifth proximal aperture is rotated 355 degrees about the longitudinal axis with respect to the first proximal aperture.

13. The humeral intramedullary nail of any one of claims 1 to 12 wherein the second distal aperture is rotated 30 degrees with respect to one of the first or third distal aperture.

14. The humeral intramedullary nail of any one of claims 3 to 13, wherein the third distal aperture is rotated 0 degrees with respect to the first distal aperture.

15. The humeral intramedullary nail of any one of claims 1 to 14, wherein the second distal aperture is rotated about the longitudinal axis with respect to the first distal aperture by at least a degree.
